# EUROPEAN PATENT APPLICATION

(11) **EP 3 789 034 A1**
(43) Date of publication of application: **10.03.2021**
(21) Application number: 20185823.0
(22) Date of filing: 01.02.2014
(51) Int. Cl.: A61K 38/18, A61K 33/42, A61P 7/06, A61P 3/00, A61P 3/02, A61P 7/08, A61P 13/12

(54) **TREATMENT OF IRON DEFICIENCY WITH SOLUBLE FERRIC PYROPHOSPHATE**

(30) Priority: 01.02.2013 US 201361759531 P
(62) Divisional of application: 14704273.3
(71) Applicant: Charak LLC, Cerritos, CA 90703 (US)
(72) Inventor: Gupta, Ajay, Cerritos, CA California 90703 (US)
(74) Representative: Lang, Christian

(57) **Abstract**

The invention provides for soluble ferric pyrophosphate in an amount effective to maintain or increase Hgb levels in the subject for use in methods of treating iron deficiency and in methods of reducing or eliminating the dose of erythropoiesis stimulating agent for increasing or maintaining hemoglobin levels in the target range in a subject suffering from anemia.

## Description

The application claims priority to U.S. Provisional Patent Application No. 61/759,531 filed February 1, 2013 which is herein incorporated by reference in its entirety.

### FIELD OF INVENTION

The invention provides for methods of treating iron deficiency, improving erythropoiesis and thereby reducing or eliminating the need for erythropoiesis stimulating agent for achieving or maintaining target hemoglobin levels in a subject suffering from anemia comprising administering soluble ferric pyrophosphate by oral or parenteral route in an amount effective to maintain or increase Hgb levels in the subject.

### BACKGROUND

Anemia is a common clinical feature of disease states characterized by concomitant inflammation such as cancer, autoimmune diseases, chronic infections and chronic kidney disease (CKD). The anemia in CKD patients is multifactorial in origin (Babitt & Lin, J. AM. Soc. Nephrol. 23: 1631, 1634, 2012). The anemia of CKD was previously largely attributed to impaired production of erythropoietin by the diseased kidneys. Treatment with recombinant human erythropoietin or other erythropoiesis stimulating agents (ESA) improves anemia partially but may be associated with adverse effects including worsening hypertension, seizures, and dialysis access clotting. In addition, ESAs may lead to increased risk of death, adverse cardiovascular events and strokes when administered to achieve hemoglobin (Hgb) levels > 11 g/dL. Therefore, it is important to detect and treat other etiological factors that lead to or worsen anemia in CKD patients, so that the minimum doses of ESAs can be used in any individual patient to treat anemia and prevent the need for blood transfusion, while minimizing the risks associated with use of ESAs.

Iron deficiency is a major contributor to anemia in CKD patients. Absolute iron deficiency with depletion of iron stores can be corrected by intravenous administration of iron. However, the efficacy of intravenous iron in the setting of anemia of inflammation as in patients with CKD is impaired, since intravenously administered iron-carbohydrate complexes get trapped in the reticulo-endothelial system (RES) by the action of hepcidin, a peptide released from the liver cells in states of inflammation (Babitt & Lin, J. AM. Soc. Nephrol. 23: 1631, 1634, 2012). This results in reticulo-endothelial block and thereby a state of functional iron deficiency.

Therefore, there is a need for methods of treating anemia which increase or maintain hemoglobin (Hgb) levels while reducing the dose of ESA administered to achieve or maintain Hgb levels.

### SUMMARY OF INVENTION

The administration of soluble ferric pyrophosphate (SFP) would overcome both absolute and functional iron deficiencies in hemodialysis dependent CKD patients (CKD-HD) patients, and could significantly reduce the amount of ESA needed to treat these CKD patients. SFP-iron directly binds to apo-transferrin, thereby delivering SFP-iron to bone marrow directly, bypassing the RES (Gupta et al. J Am Soc Nephrol 2010; 21 (Renal Week 2010 Abstract Supplement): 429A, 2010). The invention provides for method of reducing or eliminating the dose of ESA to increase or maintain Hgb levels in subjects suffering from anemia. The study described in Example 1 demonstrates that the parenteral administration of SFP, e.g. 110 µg/L of SFP administered parenterally, resulted in up to about 50% reduction in the dose ESA administered prior to the administration of SFP while maintaining or increasing Hgb levels.

The invention provides for methods of treating iron deficiency that reduces or eliminates the dose of an erythropoiesis stimulating agent (ESA) for achieving or maintaining target hemoglobin levels in a subject suffering from anemia comprising (a) administering to the subject a dose of a soluble ferric pyrophosphate composition (SFP) effective to increase hemoglobin levels of the subject, and (b) after SFP administration, (i) administering to the subject a dose of ESA that is at least 15% less than the dose of ESA administered prior to SFP administration, or (ii) discontinuing ESA administration. The dose of ESA may be administered via parenteral or oral delivery.

The invention provides for methods of reducing or eliminating the dose of an erythropoiesis stimulating agent (ESA) for maintaining hemoglobin (Hgb) levels in a subject suffering from anemia comprising (a) administering to the subject, a dose of a soluble ferric pyrophosphate composition (SFP) effective to increase Hgb levels of the subject, and (b) after SFP administration, (i) administering to the subject a dose of ESA that is at least 15% less than the dose of ESA administered prior to SFP administration, (ii) discontinuing ESA administration. The dose of ESA may be administered via parenteral or oral delivery.

The invention also provides for methods of delaying or preventing iron deficiency and delaying or preventing the need for administration of a dose of an erythropoiesis stimulating agent (ESA) for achieving or maintaining target hemoglobin levels in a subject suffering from iron loss or iron deficiency comprising (a) administering to the subject a therapeutically effective does of soluble ferric pyrophosphate composition (SFP) effective to increase hemoglobin levels of the subject, and (b) delaying or preventing the administration of ESA, wherein the subject is not currently receiving ESA to achieve or maintain target hemoglobin levels. The dose of ESA may be administered via parenteral or oral delivery.

The invention also provides for a method of delaying or preventing the need for administration an erythropoiesis stimulating agent (ESA) to increase or maintain Hgb levels in a subject suffering from anemia comprising (a) administering to the subject a dose of a soluble ferric pyrophosphate composition (SFP) effective to increase Hgb levels of the subject, and (b) delaying or preventing the administration of ESA, wherein the subject is not currently receiving ESA to maintain Hgb levels. The dose of ESA may be administered via parenteral or oral delivery.

"Oral delivery" refers to administering a therapeutic agent such as a dose of SFP by mouth leading to gastrointestinal absorption. "Parenteral delivery" refers to administering a therapeutic agent, such as a dose of SFP, by injection or infusion, such as via intravenous, subcutaneous, intramuscular, intradermal, transdermal, transbuccal, sublingual or intraperitoneal route. Parenteral delivery also includes administration via hemodialysis when added to (or in conjunction with) hemodialysis solutions, via peritoneal dialysis when added to (or in conjunction with) peritoneal dialysis solutions, or in conjunction with parenteral nutrition admixtures when added to parenteral nutrition admixture.

The invention also provides for a composition of SFP for use in treating iron deficiency while reducing or eliminating the dose of ESA for achieving or maintaining target Hgb levels in a subject suffering from anemia, wherein SFP is at a dose that is effective to increase Hgb levels of the subject and wherein after administration of the SFP composition, the dose of ESA administered to the subject is at least 15% less than the dose of ESA administered prior to SFP administration or the ESA administration is discontinued. The composition may comprise SFP in a dose form for oral and/or parenteral delivery.

The invention also provides for a composition of SFP for use in reducing or eliminating the dose of ESA for maintaining Hgb levels in a subject suffering from anemia, wherein SFP is at a dose that is effective to increase Hgb levels of the subject and wherein after administration of the SFP composition, the dose of ESA administered to the subject is at least 15% less than the dose of ESA administered prior to SFP administration or the ESA administration is discontinued. The composition may comprise SFP in a dose form for oral and/or parenteral delivery.

The invention also provides for a composition of SFP for use in delaying or preventing iron deficiency and delaying or preventing the need for administration of a dose of ESA to achieve or maintain target Hgb levels in a subject suffering from iron loss or iron deficiency, wherein SFP is at a dose that is effective to increase Hgb levels of the subject, and wherein administration of the composition results in the delay or prevention of administration of ESA in a subject that is not currently receiving ESA to achieve or maintain target Hgb levels. The composition may comprise SFP in a dose from for oral and/or parenteral delivery.

The invention also provides for a composition of SFP for use in delaying or preventing the need for administration of a dose of ESA to increase or maintain Hgb levels in a subject suffering from iron deficiency or iron loss, wherein SFP is at a dose that is effective to increase Hgb levels of the subject, and wherein administration of the composition results in the delay or prevention of administration of ESA in a subject that is not currently receiving ESA to maintain Hgb levels. The composition may comprise SFP in a dose from for oral and/or parenteral delivery.

The invention also provides for the use of a dose of SFP for the preparation of a medicament that is effective to increase Hgb levels of a subject, thereby reducing or eliminating the need for ESA for achieving or maintaining target Hgb levels in a subject suffering from anemia. The medicament may be formulated for delivery via oral and/or parenteral route. The uses of the invention include the preparation of medicaments comprising SFP wherein, after administration of said medicament to a subject suffering from anemia, (i) the dose of ESA administered to the subject is at least 15% less than the dose of ESA administered prior to administration of the medicament, or (ii) ESA administration is discontinued.

The invention also provides for the use of a dose of SFP for the preparation of a medicament that is effective to increase Hgb levels of the subject, thereby reducing or eliminating the need for ESA for maintaining Hgb levels in a subject suffering from anemia. The medicament may be formulated for delivery via oral and/or parenteral route. The uses of the invention include the preparation of medicaments comprising SFP wherein, after administration of said medicament to a subject suffering from anemia, (i) the dose of ESA administered to the subject is at least 15% less than the dose of ESA administered prior to administration of the medicament, or (ii) ESA administration is discontinued.

The invention also provides for the use of a dose of SFP for the preparation of a medicament that is effective to increase Hgb levels of a subject, thereby delaying or preventing iron deficiency and delaying or preventing the need for ESA for maintaining Hgb levels in a subject suffering from iron loss or iron deficiency. The medicament may be formulated for delivery via oral and/or parenteral route.

The invention also provides for methods of treating iron deficiency with and without anemia. These methods comprise administering a therapeutically effective dose of SFP. In patients with iron deficiency anemia, the therapeutically effective dose of SFP will increase markers of iron status such as serum iron, transferrin saturation, reticulocyte hemoglobin, serum ferritin, reticulocyte count, and whole blood hemoglobin while reducing or eliminating the need for ESAs if the patient is a candidate for ESA administration. Furthermore, the therapeutically effective dose will reduce or eliminate the need for transfusion of whole blood or packed red blood cell or blood substitutes. In non-anemic patients with iron deficiency, as in patients with iron deficiency anemia, the therapeutically effective dose of SFP will reduce fatigue, increase physical and cognitive ability, and improve exercise tolerance. If an iron deficient patient, with or without anemia, suffers from restless leg syndrome (RLS) the therapeutically effective dose of SFP will reduce or abolish the clinical manifestations of RLS.

Anemic subjects have reduced Hgb levels and the methods of the invention administer a dose of SFP effective to increases Hgb levels in the anemic subject. For example, the dose of SFP administered increases Hgb level to that which is sufficient to adequately oxygenate the subject's tissues or provides improved oxygenation of the anemic subject's tissues. Preferably, the dose of SFP administered increases or maintains the hemoglobin level of the subject at 9-10 g/dL or greater thereby reducing the need for blood transfusions, reducing fatigue, improving physical and cognitive functioning, improving cardiovascular function, improving exercise tolerance and enhancing quality of life. For example, Hgb levels are increased to or maintained at a target level ranging from 9to 10 g/dL, at a target level ranging from 9 g/dL to 12 g/dL, at a target level ranging from 10 g/dL to 12 g/dL, at a target level ranging from 9 g/dL to 14 g/dL, at a target level ranging from 10 g/dL to 14 g/dL, or at a target level ranging from 12 g/dL to 14 g/dL.

In addition, the invention provides for methods of administering a dose of SFP effective to increase or maintain Hgb at a target level of at least about 9 g/dL, of at least about 10 g/dL, of at least about 11 g/dL, of at least about 12 g/dL, of at least about 13 g/dL of at least about 14 g/dL, of about 9-11 g/dL, of about 9-12 g/dL or at about 9-14 g/dL.

In an aspect of the invention, the dose of SFP for any of the preceding methods is administered via hemodialysate at a iron dose ranging from 90 µg/L dialysate to 150 µg/L dialysate, or at a dose ranging from 90 µg/L dialysate to 140 µg/L dialysate, or at a dose ranging from 90 µg/L dialysate to 130 µg/L dialysate, or at a dose ranging from 90 µg/L dialysate to 120 µg/L dialysate, or at a dose ranging from 90 µg/L dialysate to 110 µg/L dialysate, or at a dose ranging from 90 µg/L dialysate to 105 µg/L dialysate, or at a dose ranging from 105 µg/L dialysate to 115 µg/L dialysate, or at a dose ranging from 105 µg/L dialysate to 110 µg/L dialysate, or at a dose ranging from 105 µg/L dialysate to 120 µg/L dialysate, or at a dose ranging from 105 µg/L dialysate to 130 µg/L dialysate, or at a dose ranging from 105 µg/L dialysate to 140 µg/L dialysate, or at a dose ranging from 105 µg/L dialysate to 150 µg/L dialysate, or at a dose ranging from 110 µg/L dialysate to 150 µg/L dialysate, or at a dose ranging from 110 µg/L dialysate to 140 µg/L dialysate, or at a dose ranging from 110 µg/L dialysate to 130 µg/L dialysate, or at a dose ranging from 110 µg/L dialysate to 120 µg/L dialysate, or at a dose ranging from 110 µg/L dialysate to 115 µg/L dialysate, or at a dose ranging from 112 µg/L dialysate to 150 µg/L dialysate, or at a dose ranging from 112 µg/L dialysate to 140 µg/L dialysate, or at a dose ranging from 112 µg/L dialysate to 130 µg/L dialysate, or at a dose ranging from 112 µg/L dialysate to 120 µg/L dialysate, or at a dose ranging from 112 µg/L dialysate to 118 µg/L dialysate, or at a dose ranging from 112 µg/L dialysate to 115 µg/L dialysate, or at a dose ranging from 115 µg/L dialysate to 150 µg/L dialysate, or at a dose ranging from 115 µg/L dialysate to 140 µg/L dialysate, or at a dose ranging from 115 µg/L dialysate to 130 µg/L dialysate, or at a dose ranging from 115 µg/L dialysate to 120 µg/L dialysate, or at a dose ranging from 120 µg/L dialysate to 150 µg/L dialysate, or at a dose ranging from 120 µg/L dialysate to 140 µg/L dialysate, or at a dose ranging from 120 µg/L dialysate to 130 µg/L dialysate, or at a dose ranging from 120 µg/L dialysate to 125 µg/L dialysate, or at a dose ranging from 130 µg/L dialysate to 150 µg/L dialysate, or at a dose ranging from 130 µg/L dialysate to 140 µg/L dialysate, or at a dose ranging from 140 µg/L dialysate to 150 µg/L dialysate.

In an exemplary aspect of the invention, the dose of SFP is administered at a dose of 110 µg or 2 µmoles SFP- iron per liter of hemodialysate. In addition, the invention provides for methods wherein the dose of SFP iron is administered via dialysate at a dose of about 105 µg/L dialysate, about 106 µg/L dialysate, about 107 µg/L dialysate, about 108 µg/L dialysate, about 109 µg/L dialysate, about 110 µg/L dialysate, about 111 µg/L dialysate or about 112 µg/L dialysate.

In one aspect of the invention, the dose of SFP for any of the preceding methods is administered via infusion at a dose ranging from 2.4 mg to 48 mg per day at a rate of 0.1 to 2 mg per hour m. In another aspect of the invention, the dose of SFP is administered via intravenous injection at a dose ranging from 2.4 mg to 48 mg per day at a rate of 0.1 to 2 mg per hour. In addition, the invention provides for any of the preceding methods wherein, the dose of SFP is administered into the circulation at a dose ranging from 2.4 mg to 48 mg per day at a rate of 0.1 to 2 mg per hour. For any of these methods, the dose administered to the subject is based on the bioavailability of SFP using the specific route of administration.

Additional exemplary dose ranges for administering SFP-iron via infusion, intravenous injection or delivery into the circulation include a dose ranging from 5 mg to 48 mg per day at a rate of 0.1 to 2 mg per hour, or at a dose ranging from 10 mg to 48 mg per day at a rate of .01 to 2 mg per hour, or at a dose ranging from 20 mg to 48 mg per day at a rate of .01 to 2 mg per hour, or at a dose ranging from 30 mg to 48 mg per day at a rate of .01 to 2 mg per hour, or at a dose ranging from 40 mg to 48 mg per day at a rate of .01 to 2 mg per hour, or a dose ranging from 2.4 mg to 48 mg per day at a rate of 1 to 2 mg per hour, or a dose ranging from 5 mg to 48 mg per day at a rate of 1 to 2 mg per hour, or at a dose ranging from 10 mg to 48 mg per day at a rate of 1 to 2 mg per hour, or at a dose ranging from 20 mg to 48 mg per day at a rate of 1 to 2 mg per hour, or at a dose ranging from 30 mg to 48 mg per day at a rate of 1 to 2 mg per hour, or at a dose ranging from 40 mg to 48 mg per day at a rate of 1 to 2 mg per hour, or a dose ranging from 2.4 mg to 48 mg per day at a rate of 0.5 to 1 mg per hour, or a dose ranging from 5 mg to 48 mg per day at a rate of 0.5 to 1 mg per hour, or at a dose ranging from 10 mg to 48 mg per day at a rate of 0.5 to 1 mg per hour, or at a dose ranging from 20 mg to 48 mg per day at a rate of 0.5 to 1 mg per hour, or at a dose ranging from 30 mg to 48 mg per day at a rate of. 0.5 to 1 mg per hour, or at a dose ranging from 40 mg to 48 mg per day at a rate of 0.5 to 1 mg per hour, a dose ranging from 2.4 mg to 40 mg per day at a rate of 0.1 to 2 mg per hour, or a dose ranging from 5 mg to 40 mg per day at a rate of 0.1 to 2 mg per hour, or at a dose ranging from 10 mg to 40 mg per day at a rate of .01 to 2 mg per hour, or at a dose ranging from 20 mg to 40 mg per day at a rate of .01 to 2 mg per hour, or at a dose ranging from 30 mg to 40 mg per day at a rate of .01 to 2 mg per hour, or at a dose ranging from 40 mg to 40 mg per day at a rate of .01 to 2 mg per hour, or a dose ranging from 2.4 mg to 40 mg per day at a rate of 1 to 2 mg per hour, or a dose ranging from 5 mg to 40 mg per day at a rate of 1 to 2 mg per hour, or at a dose ranging from 10 mg to 40 mg per day at a rate of 1 to 2 mg per hour, or at a dose ranging from 20 mg to 40 mg per day at a rate of 1 to 2 mg per hour, or at a dose ranging from 30 mg to 40 mg per day at a rate of 1 to 2 mg per hour, a dose ranging from 2.4 mg to 40 mg per day at a rate of 0.5 to1 mg per hour, or a dose ranging from5 mg to 40 mg per day at a rate of 0.5 to 1 mg per hour, or at a dose ranging from 10 mg to 40 mg per day at a rate of 0.5 to 1 mg per hour, or at a dose ranging from 20 mg to 40 mg per day at a rate of 0.5 to 1 mg per hour, or at a dose ranging from 30 mg to 40 mg per day at a rate of. 0.5 to 1 mg per hour, a dose ranging from 2.4 mg to 30 mg per day at a rate of 0.1 to 2 mg per hour, or a dose ranging from 5 mg to 30 mg per day at a rate of 0.1 to 2 mg per hour, or at a dose ranging from 10 mg to 30 mg per day at a rate of .01 to 2 mg per hour, or at a dose ranging from 20 mg to 30 mg per day at a rate of .01 to 2 mg per hour, or a dose ranging from 2.4 mg to 30 mg per day at a rate of 1 to2 mg per hour, or a dose ranging from 5 mg to 30 mg per day at a rate of 1 to 2 mg per hour, or at a dose ranging from 10 mg to 30mg per day at a rate of 1 to 2 mg per hour, or at a dose ranging from 20 mg to 30 mg per day at a rate of 1 to 2 mg per hour, a dose ranging from 2.4 mg to 30 mg per day at a rate of 0.5 to 1 mg per hour, or a dose ranging from 5 mg to 30 mg per day at a rate of 0.5 to 1 mg per hour, or at a dose ranging from 10 mg to 30 mg per day at a rate of 0.5 to 1 mg per hour, or at a dose ranging from 20 mg to 30 mg per day at a rate of0.5 to 1 mg per hour, a dose ranging from 2.4 mg to 20 mg per day at a rate of 0.1 to 2 mg per hour, or a dose ranging from 5 mg to 20 mg per day at a rate of 0.1 to 2 mg per hour, or at a dose ranging from 10 mg to 20 mg per day at a rate of .01 to 2 mg per hour, or a dose ranging from 2.4 mg to 20 mg per day at a rate of 1 to 2 mg per hour, or a dose ranging from 5 mg to20 mg per day at a rate of 1 to 2 mg per hour, or at a dose ranging from 10 mg to 20 mg per day at a rate of 1 to 2 mg per hour, a dose ranging from 2.4 mg to 20 mg per day at a rate of 0.5 to 1 mg per hour, or a dose ranging from5 mg to 20 mg per day at a rate of 0.5 to 1 mg per hour, or at a dose ranging from 10 mg to 20 mg per day at a rate of 0.5 to 1 mg per hour, a dose ranging from 5 mg to 10 mg per day at a rate of 0.1 to 2 mg per hour, a dose ranging from5 mg to 10 mg per day at a rate of 0.1 to 2 mg per hour, or a dose ranging from 2.4 mg to 10 mg per day at a rate of 1 to 2 mg per hour, or a dose ranging from 5 mg to 10 mg per day at a rate of 1 to 2 mg per hour, or a dose ranging from 2.4 mg to 10 mg per day at a rate of 0.5 to 1 mg per hour, a dose ranging from5 mg to 10 mg per day at a rate of 0.5 to 1 mg per hour, or a dose ranging from2.4 mg to 5 mg per day at a rate of 0.1 to 2 mg per hour, or a dose ranging from 2.4 mg to 5 mg per day at a rate of 0.5 to 1 mg per hour, a dose ranging from 2.4 mg to 5 mg per day at a rate of 1 to 2 mg per hour.

In any of the preceding methods, the dose of ESA administered to the subject after SFP administration is at least 15% less than the dose of ESA administered prior to SFP administration, or is at least 20% less than the dose of ESA administered prior to SFP administration, or is at least 25% less than the dose of ESA administered prior to SFP administration, or is at least 33% less than the dose of ESA administered prior to SFP administration, or is at least 40% less than the dose of ESA administered prior to SFP administration, or is at least 42% less than the dose of ESA administered prior to SFP administration, or is at least 45% less than the dose of ESA administered prior to SFP administration, or is at least 47% less than the dose of ESA administered prior to SFP administration, or is at least 50% less than the dose of ESA administered prior to SFP administration, or is at least 51% less than the dose of ESA administered prior to SFP.

In addition, in any of the preceding methods, the dose of ESA administered to the subject after SFP administration ranges from 15% to 50% less than the dose of ESA administered prior to SFP administration, ranges from 15% to 45% less than the dose of ESA administered prior to SFP administration, ranges from 15% to 40% less than the dose of ESA administered prior to SFP administration, ranges from 15% to 35% less than the dose of ESA administered prior to SFP administration, ranges from 15% to 30% less than the dose of ESA administered prior to SFP administration, ranges from 15% to 25% less than the dose of ESA administered prior to SFP administration, ranges from 15% to 20% less than the dose of ESA administered prior to SFP administration, ranges from 15% to 18% less than the dose of ESA administered prior to SFP administration, ranges from 20% to 50% less than the dose of ESA administered prior to SFP administration, ranges from 20% to 45% less than the dose of ESA administered prior to SFP administration, ranges from 20% to 40% less than the dose of ESA administered prior to SFP administration, ranges from 20% to 35% less than the dose of ESA administered prior to SFP administration, ranges from 20% to 30% less than the dose of ESA administered prior to SFP administration, ranges from 20% to 25% less than the dose of ESA administered prior to SFP administration, ranges from 25% to 45% less than the dose of ESA administered prior to SFP administration, ranges from 25% to 40% less than the dose of ESA administered prior to SFP administration, ranges from 25% to 35% less than the dose of ESA administered prior to SFP administration, ranges from 25% to 30% less than the dose of ESA administered prior to SFP administration, ranges from 30% to 50% less than the dose of ESA administered prior to SFP administration, ranges from 30% to 45% less than the dose of ESA administered prior to SFP administration, ranges from 30% to 40% less than the dose of ESA administered prior to SFP administration, ranges from 30% to 35% less than the dose of ESA administered prior to SFP administration, ranges from 35% to 50% less than the dose of ESA administered prior to SFP administration, ranges from 35% to 45% less than the dose of ESA administered prior to SFP administration, ranges from 40% to 50% less than the dose of ESA administered prior to SFP administration, ranges from 40% to 45% less than the dose of ESA administered prior to SFP administration, ranges from 40% to 42% less than the dose of ESA administered prior to SFP administration, ranges from 45% to 50% less than the dose of ESA administered prior to SFP administration, ranges from 27% to 35% less than the dose of ESA administered prior to SFP administration, ranges from 27% to 40% less than the dose of ESA administered prior to SFP administration, ranges from 32% to 35% less than the dose of ESA administered prior to SFP administration, ranges from 32% to 38% less than the dose of ESA administered prior to SFP administration, or ranges from 40% to 51% less than the dose of ESA administered prior to the SFP administration.

In another embodiment, the invention provides for methods of increasing Hgb levels in subjects suffering from anemia comprising administering to the subject 110 µg SFP iron via per liter of hemodialysate. These methods may further comprise administering a dose of ESA, after SFP administration, that is at less than the dose of ESA administered prior to SFP administration wherein the subject is receiving erythropoiesis stimulating agent (ESA) to maintain hemoglobin levels prior to administration of SFP, such as 15%, 20%, or 25% less than the dose of ESA administered prior to SFP administration. For example, the method of the invention increases or maintains the level of Hgb in an anemic subject to at least about 9 g/dL, or to at least about 10 g/dL, or to at least about 12 g/dL, or to at least about 14 g/dL. The methods of the invention increase or maintain the level of Hgb in an anemic subject so that the need for transfusions or whole blood, packed red blood cells or blood substitutes is reduced or eliminated.

In another embodiment, the invention provides for methods of increasing Hgb levels in subjects suffering from anemia comprising administering to the subject 2.4 to 48 mg per day at a rate of 0.1 to 2 mg per hour SFP into the circulation of the subject by a route selected from the group consisting of oral, intramuscular, subcutaneous, intravenous, transbuccal, intradermal, transdermal, sublingual or intraperitoneal, in conjunction with the dialysis solutions in patients with kidney disease undergoing hemodialysis or peritoneal dialysis. In addition, the SFP may be administered into the circulation of the subject in conjunction with other drugs such as heparin or parenteral nutrition admixtures, and the dose administered to the patient is based on the bioavailability of SFP using the specific route of administration For example, the subject is administered 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg per day at a rate of 0.1 to 2 mg per hour via intravenous injection or infusion, or 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg per day at a rate of 0.5 to 1 mg per hour via intravenous injection or infusion, or 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg per day at a rate of 1 to 2 mg per hour via intravenous injection or infusion. These methods may further comprise administering a dose of ESA, after SFP administration, that is at less than the dose of ESA administered prior to SFP administration wherein the subject is receiving erythropoiesis stimulating agent (ESA) to maintain hemoglobin levels prior to administration of SFP, such as 15%, 20%, or 25% less than the dose of ESA administered prior to SFP administration. For example, the method of the invention increases or maintains the level of Hgb in an anemic subject to at least about 9 g/dL, or to at least about 10 g/dL, or to at least about 12 g/dL, or to at least about 14 g/dL. The methods of the invention increase or maintain the level of Hgb in an anemic subject so that the need for blood transfusions is reduced or eliminated.

The invention also provides for use of SFP for the preparation of a medicament for increasing Hgb levels in a subject suffering from anemia wherein the medicament comprises about 110 µg/L of SFP and the medicament is administered via dialysate. The use of this medicament may also reduce the dose of ESA administered prior to SFP administration wherein the subject is receiving erythropoiesis stimulating agent (ESA) to maintain hemoglobin levels prior to administration of the medicament, such as 15%, 20%, or 25% less than the dose of ESA administered prior to administration of the medicament.

The invention also provides for use of SFP for the preparation of a medicament for increasing Hgb levels in a subject suffering from anemia wherein the medicament comprises 2.4 to 48 move SFP per day at a rate of 0.1 to 2 mg per hour and the medicament is administered into the circulation of the subject by a route selected from the group consisting of oral, intramuscular, subcutaneous, intravenous, intradermal, transdermal, transbuccal, sublingual, intraperitoneal, in conjunction with the dialysis solutions in patients with kidney disease undergoing hemodialysis or peritoneal dialysis, in conjunction with heparin or in conjunction with parenteral nutrition admixtures, and the dose administered to the patient is based on the bioavailability of SFP using the specific route of administration. For example, the medicament comprises 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg administered per day at a rate of 0.1 to 2 mg per hour via intravenous injection or infusion, or 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg administer per day at a rate of 0.5 to 1 mg per hour via intravenous injection or infusion, or 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg administered per day at a rate of 1 to 2 mg per hour via intravenous injection or infusion. The use of this medicament may also reduce the dose of ESA administered prior to SFP administration wherein the subject is receiving erythropoiesis stimulating agent (ESA) to maintain hemoglobin levels prior to administration of the medicament, such as 15%, 20%, or 25% less than the dose of ESA administered prior to administration of the medicament.

The invention also provides for a composition of SFP for increasing Hgb levels in a subject suffering from anemia comprising about 110 µg SFP-iron/L and the medicament is administered via dialysate. The use of this medicament may also reduce the dose of ESA administered prior to SFP administration wherein the subject is receiving erythropoiesis stimulating agent (ESA) to maintain hemoglobin levels prior to administration of the medicament, such as 15%, 20%, or 25% less than the dose of ESA administered prior to administration of the medicament.

The invention also provides for a composition of SFP for increasing Hgb levels in a subject suffering from anemia comprising about 2.4 m g to 48 mg SPF administered per day at a rate of 0.1 to 2 mg per hour and the medicament is administered into the circulation of the subject by a route selected from the group consisting of oral, intramuscular, subcutaneous, intravenous, intradermal, transdermal, transbuccal, sublingual, intraperitoneal, in conjunction with the dialysis solutions in patients with kidney disease undergoing hemodialysis or peritoneal dialysis, in conjunction with heparin or in conjunction with parenteral nutrition admixtures , and the dose administered to the patient is based on the bioavailability of SFP using the specific route of administration. For example, the medicament comprises 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg administered per day at a rate of 0.1 to 2 mg per hour via intravenous injection or infusion, or 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg administered per day at a rate of 0.5 to 1 mg per hour via intravenous injection or infusion, or 2.4 to 40 mg, 2.4 to 30 mg, 2.4 to 20 mg, 2.4 to 10 mg, 2.4 to 5 mg, 5 to 40 mg, 5 to 30 mg, 5 to 20 mg, 5 to 10 mg, 10 to 40 mg, 10 to 30 mg, 10 to 20 mg, 20 to 40 mg, 20 to 30 mg, 30 to 40 mg administered per day at a rate of 1 to 2 mg per hour via intravenous injection or infusion.

The invention provides for any of the preceding methods wherein the SFP is in the form of a citrate chelate, i.e. iron chelated with citrate and pyrophosphate. In addition, in any of the preceding methods, the SFP comprises iron in an amount of 7% to 11% by weight, citrate in an amount of at least 14% by weight, and pyrophosphate in an amount of at least 10% by weight. In one embodiment, the SFP may comprise citrate in the amount of 14% to 30% by weight. In addition, the SFP may also comprise phosphate in an amount of 2% or less, or in an amount of 1% or less.

Furthermore, the invention provides for any of the preceding methods wherein the SFP is administered via dialysate and the dose of intravenously administered iron after SFP administration is at least 10% less than the dose of intravenously administered iron prior to SFP administration, or is at least 12.5% less than the dose of intravenously administered iron prior to SFP administration, or is at least 25% less than the dose of intravenously administered iron prior to SFP administration, or is at least 30% less than the dose of intravenously administered iron prior to SFP administration or is at least 33% less than the dose of intravenously administered iron prior to SFP administration, or is at least 50% less than the dose of intravenously administered iron prior to SFP administration, or is at least 75% less than the dose of intravenously administered iron prior to SFP administration, or is 100% less than the dose of intravenously administered iron prior to SFP administration.

The invention also provides for any of the preceding methods wherein the dose of intravenously administered iron after SFP administration ranges from at least about 10% less to about 50% less than the dose of intravenously administered iron prior to SFP administration, or ranges from at least about 10% less to about 25% less than the dose of intravenously administered iron prior to SFP administration, or ranges from at least about 25% less to about 50% less than the dose of intravenously administered iron prior to SFP administration, or ranges from at least about 50% less to about 75% less than the dose of intravenously administered iron prior to SFP administration, or ranges from at least about 75% less to about 100% less than the dose of intravenously administered iron prior to SFP administration, or ranges from about 10% less to about 100% less than the dose of intravenously administered iron prior to SFP administration, or from about 25% less to about 100% less than the dose of intravenously administered iron prior to SFP administration.

In any of the preceding methods of the invention, the subject may be suffering from chronic kidney disease, optionally stage II, III, IV or V.

In addition, the invention provides for any of the preceding methods wherein the subject is undergoing hemodialysis.

The invention also provides for any of the preceding methods wherein the subject is suffering from anemia of inflammation.

The invention also provides for any of the preceding methods wherein the subject is suffering from infection, optionally chronic infection.

Furthermore, the invention provides for any of the preceding methods wherein the subject is suffering from cancer, heart failure, autoimmune disease, sickle cell disease, thalassemia, blood loss, transfusion reaction, diabetes, vitamin B12 deficiency, collagen vascular disease, Shwachman syndrome, thrombocytopenic purpura, Celiac disease, endocrine deficiency state such as hypothyroidism or Addison's disease, autoimmune disease such as Crohn's Disease, systemic lupus erythematosis, rheumatoid arthritis or juvenile rheumatoid arthritis, ulcerative colitis immune disorders such as eosinophilic fasciitis, hypoimmunoglobulinemia, or thymoma/thymic carcinoma, graft vs. host disease, preleukemia, Nonhematologic syndrome (Down's, Dubowwitz, Seckel), Felty syndrome, hemolytic uremic syndrome, myelodysplasic syndrome, nocturnal paroxysmal hemoglobinuria, osteomyelofibrosis, pancytopenia, pure red-cell aplasia, Schoenlein-Henoch purpura, malaria, protein starvation, menorrhagia, systemic sclerosis, liver cirrhosis, hypometabolic states, congestive heart failure, chronic infections such as HIV/ AIDS, tuberculosis, oseomyelitis, hepatitis B, hepatitis C, Epstein-bar virus or parvovirus, T cell leukemia virus, bacterial overgrowth syndrome, fungal or parasitic infections, and/or red cell membrane disorders such as hereditary spherocytosis, hereditary elliptocytosis, heriditray pyrpoikilocytosis, hereditary stomatocytosis, red cell enzyme defects, hypersplenism, immune hemolysis or paroxysmal nocturnal hemoglobinuria.

In addition, the invention provides for any of the preceding methods wherein the anemia is due to overt iron deficiency with depleted iron stores or a functional iron deficiency.

The invention provides for any of the preceding methods wherein SFP is administered during hemodialysis within the hemodialysate solution. In addition, the invention provides for any of the preceding methods wherein SFP is administered during peritoneal dialysis within the peritoneal dialysis solution or wherein SFP is administered with parenteral nutrition within parenteral nutrition admixture. The invention also provides for any of the preceding methods wherein the SFP administration is by oral, intravenous, intramuscular, subcutaneous, transbuccal, sublingual, intraperitoneal, intradermal or transdermal routes, or in conjunction with the dialysis solutions in patients with kidney disease undergoing hemodialysis or peritoneal dialysis.

The invention also provides for any of the preceding methods wherein SFP is administered at a therapeutically effective dose that i) increases at least one marker of iron status selected from the group consisting of serum iron, transferrin saturation, reticulocyte hemoglobin, serum ferritin, reticulocyte count, and whole blood hemoglobin and ii) decreases or eliminates the need for ESA administration to achieve or maintain target hemoglobin levels, or the need for transfusion of whole blood, packed red blood cell or blood substitutes. In addition, when any of the preceding methods are carried out in a subject is suffering from non-anemic iron deficiency and administration of the therapeutically effective dose of SFP reduces fatigue, increases physical and cognitive ability, or improves exercise tolerance in the subj ect.

In addition, the invention provides for any of the preceding methods wherein SFP is administered in a therapeutically effective dose that will reduce or abolish the clinical manifestations of restless leg syndrome associated with iron deficiency.

Doses include, for example, about 1-50 mg SFP-iron per day at a rate of 0.1 to 2 mg per hour, given by any administration route, or about 2 to 48 mg per day, or 2 to 25 mg per day, or 2 to 10 mg per day, or 3 to 48 mg per day, or 3 to 25 mg per day, or 3 to 10 mg per day, or 4 to 48 or 4 to 25 mg per day, or 4 to 30 mg per day, or 4 to 10 mg per day, or 5 to 50 mg per day, 10 to 50 mg per day, 5 to 45 mg per day, 10 to 45 mg per day, 5 to 25 mg per day, 5 to 10 mg per day, 10 to 25 mg per day, 10 to 30 mg per day. Any of these doses may be administered at a rate of 0.1 to 2 mg per hour, or at a rate of 0.5 to 1 mg per hour, or at a rate of1 to 2 mg per hour.

### BRIEF DESCRIPTION OF DRAWING

**Figure** 1 shows the cumulative IV iron administration to the patients participating in the clinical study described in Example 1. The estimated mean weekly dose of IV iron was 21.5 mg for SFP compared to 32.6 mg for placebo, a 34% reduction.
**Figure** 2 shows the mean Hgb level by month for the patients participating in the clinical study described in Example 1.
**Figure** 3 shows the cumulative ESA dose administered to the patients participating in the clinical study described in Example 1.

### DETAILED DESCRIPTION

Due to the adverse side effects associated with ESA therapy in anemic subjects, the lowest possible dose of ESA should be administered that will reduce or eliminate the need for blood transfusions. Furthermore, anemia of chronic kidney disease (CKD) is resistant to ESA treatment in 10-20% of patients (Babbitt and Lin, J Am Soc Nephrol 23: 1631-1634, 2012). Thus, the invention provides for methods of reducing ESA dose or eliminating the need for ESA treatment while achieving or maintaining target Hgb levels in a subject suffering from anemia, by administering a therapeutically effective dose of SFP. The clinical study described in Example 1 demonstrates that the magnitude of ESA reduction by SFP exceeds that seen with majority of other ESA-sparing interventions such as intravenous administration of carnitine, ascorbic acid or iron-carbohydrate complexes. Large doses of intravenously administered iron-carbohydrate complexes can also increase Hgb and reduce ESA need but lead to marked increase in tissue iron stores and inflammation as suggested by a marked increase in serum ferritin level (Besarab et al. J. Am, Soc. Nephrol. 11: 530-538, 2000). On the other hand, studies have shown that doses of SFP iron ranging from 100 to 120 µg per liter of hemodialysate maintain but do not increase serum ferritin levels (Gupta et al. J Am Soc Nephrol 2010; 21 (Renal Week 2010 Abstract Supplement): 429A 2010).

Therefore, the experimental evidence provided herein demonstrates highly significant ESA sparing by SFP in CKD-HD patients without an increase in tissue iron stores or inflammation. This highly unexpected result is likely due to the unique mode of action of SFP. It is now increasingly recognized that a major contributor to anemia in CKD patients is the anemia of inflammation that is due to disordered iron homeostasis largely mediated by the peptide hepcidin. Hepcidin excess likely accounts for impaired dietary iron absorption and impaired release of iron from reticuloendothelial iron stores. Produced by the liver and secreted into circulation, hepcidin binds and induces degradation of ferroportin, the iron exporter that is present on duodenal enterocytes, reticuloendothelial macrophages, and hepatocytes, thereby inhibiting iron entry into plasma. Inflammatory cytokines directly induce hepcidin transcription, presumably as a mechanism to sequester iron from invading pathogens, leading to the iron sequestration, hypoferremia, and anemia that are hallmarks of many chronic diseases including CKD. In CKD patients with hepcidin excess, large intravenous doses of iron would be predicted to have limited effectiveness because much of the iron is rapidly taken up by the liver and sequestered, and the remainder that is incorporated into red blood would be recycled ineffectively. In addition intravenous iron further increases hepcidin levels and worsens this phenomenon (Babitt & Lin, J. AM. Soc. Nephrol. 23: 1631, 1634, 2012). Administration of SFP appears to supply iron without worsening the anemia of inflammation. Without being bound by theory, SFP iron when delivered directly into the circulation by any parenteral route binds directly to the circulating iron carrier protein apotransferrin thereby forming monoferric or diferric transferrin, which then delivers iron directly to the red blood cell precursors in the bone marrow, bypassing processing by the reticulo-endothelial macrophages and hepatocytes.

It is expected that these findings apply to SFP delivered by any parenteral route. Parenteral routes for effective SFP delivery include but are not limited to intravenous, intramuscular, subcutaneous, intradermal, transdermal, transbuccal, sublingual, via hemodialysis when added to hemodialysis solutions, via peritoneal dialysis when added to peritoneal dialysis solutions, or in conjunction with parenteral nutrition admixtures when added to parenteral nutrition admixture.

### Erythropoiesis Stimulating Agents

Erythropoiesis is the generation of red blood cells (RBC) in the bone marrow. The term "erythropoiesis" is used herein to describe the process of proliferation and differentiation of hematopoietic stem cells (HSCs) and hematopoietic progenitor cells, leading to the production of mature red blood cells. "Erythropoiesis stimulating agents" (ESA) are agents that are capable of intimating and stimulating new red blood cell production.

Erythropoietin (EPO) is a circulating glycosylated protein hormone (34 KD) that is the primary regulator of RBC formation. Endogenous EPO is produced in amounts that correspond to the concentration of O₂ in the blood and is synthesized primarily in the kidney, although it is also made at lower levels in other tissues such as liver and brain.

The term "ESA" applies to all agents that augment erythropoiesis through direct or indirect action on the erythropoietin receptor. ESA includes endogenous human erythropoietin (GenBank Accession No. AAA52400; Lin et al. (1985) Proc. Natl. Acad. Sci. USA 82:7580-7584) and recombinant erythropoietin and erythropoietin-like substances such as EPOETIN products under the trade names of EPOGEN™ (Amgen, Inc., Thousand Oaks, Calif.), EPREX™ (Janssen-Cilag. Ortho Biologics LLC) and NEORECORMON™ (Roche), ARANESP human recombinant erythropoietin (Amgen), PROCRIT™ (Ortho Biotech Products, L.P., Raritan N.J.) and MIRCERA (Methoxy polyethylene glycol-epoetin beta; Roche), Peginesatide or Omontys® (Affymax Inc., Palo Alto, CA).

ESA also includes water-soluble salts of transition metals such as manganese (Mn), cobalt (Co), and nickel (Ni); and also include titanium (Ti), vanadium (V), and chromium (Cr). Most, if not all, water-soluble salts of these transition metals are believed to stimulate red cell production when administered in suitable concentration *in vivo.* The salts thus include halides and most preferably chloride salts; carbonate and bicarbonate salts; sulfides, sulfites, and sulfates; nitrogen atom containing salts; oxides; and other conventional salt formats and formulations generally biocompatible and useful *in vivo.*

The daily doses administered to subjects in need may vary for the respective ESAs. It should be noted that doses for individual patients and patient groups will often differ from the daily defined dose and will necessarily have to be based on individual characteristics (e.g. age and weight) and pharmacokinetic considerations.

ESA are commonly used to treat anemia and are administered to subjects undergoing hemodialysis. Despite the positive effects ESA have on number of red blood cells and increasing Hgb levels, ESA treatment has many adverse effects and the FDA recommends administering the lowest dose of ESA sufficient to reduce or avoid the need for RBC transfusions.

### Hemoglobin

The methods of the invention administer a dose of SFP effective to increase hemoglobin (Hgb) levels in the subject. Hgb is a spheroidal protein that consists of four subunits, two pairs of identical polypeptide chains, each with a cleft or pocket on its exterior. The cleft contains a heme or iron-protoporphyrin group that is the site of oxygen uptake and release. A primary role of Hgb in RBCs is to carry O₂ to O₂-dependent tissues. Hgb found in RBCs is a tetrameric haem iron-containing protein. Anemic subjects have reduced Hgb levels and the methods of the invention comprise administering an effective dose of SFP to increase Hgb in subjects suffering from anemia. An effective dose of SFP increases Hgb levels and preferably the Hgb levels are increased to a level that is sufficient to adequately oxygenate the subject's tissues and/or reduce the need for blood transfusion. An exemplary sufficient Hgb in level for an anemic subject is at least 9.5 g/dL. Normal Hgb levels for a human adult male range from 14 to 18 g/dL and for adult human women range from 12.0 to 16 g/dL. However, the target Hgb levels in a CKD patient receiving ESA is 9-11 g/dL.

Hemoglobin levels are determined as g/dL of blood of the subject. Hemoglobin levels may be measured using any method known in the art. For example, hemoglobin levels may be measuring using photometric detection of cyanmetahemoglobin, photmetic detection of azide metahemoglobin, or a peroxidase method (e.g. Crosby-Furth method) to name a few.

### SFP Compositions

Ferric pyrophosphate (Fe₄ 0₂i P₆) has a molecular weight of 745.25. It has been used as a catalyst, in fireproofing synthetic fibers and in corrosion preventing pigments.

Soluble ferric pyrophosphate (alternatively, "ferric pyrophosphate, soluble" or SFP) is an iron preparation of uncertain composition. No definite formula for its constitution is known. In general, it is described as "a mixture of ferric pyrophosphate and sodium citrate" or "a mixture of four salts (ferric and sodium pyrophosphates and ferric and sodium citrates)" or "ferric pyrophosphate that has been rendered soluble by sodium citrate." Soluble ferric pyrophosphate is known to have the properties described in Table 1.

**Table 1 - Properties of Conventional Ferric Pyrophosphate, Soluble**

| **Parameter** | **Observation** |
|---|---|
| Chemical Name | 1,2,3-Propanetricarboxylic acid, 2-hydroxy-, iron(3+) sodium salt |
| | (1:1:1), mixture with iron(3+) diphosphate |
| CAS Registry No. | 1332-96-3 |
| Appearance | Solid (may be plates, powder, or pearls, depending on the manufacturer |
| Color | Yellow-green to apple-green |
| Iron Content | 10.5% to 12.5% |
| Solubility in water | Exceeds 1 gram per mL |
| pH of a 5% solution | 5-7 |

The invention provides for methods of administering SFP via parenteral delivery to anemic subjects. US Patent No. 6779468 describes parenteral administration of SFP, US Patent No. 6689275, describes compositions comprising SFP solutions, and US Patent No. 7,816,404 describes water-soluble SFP citrate chelate compositions, all of these US patents are incorporated by reference herein in their entirety.

Soluble ferric pyrophosphate may be obtained commercially. Food grade soluble ferric pyrophosphate (FCC-SFP) is an apple-green solid containing from about 10.5% to about 12.5% iron. According to the manufacturers, soluble ferric pyrophosphate is stable for as long as three years provided that it is protected against exposure to air and light. Analysis of food grade soluble ferric pyrophosphates has shown that typical conventional preparations contain iron, pyrophosphate anion, citrate anion, phosphate anion, sulfate anion, and sodium (Table 2).

**Table 2 Composition of food grade soluble ferric pyrophosphates (FCC-SFP)**

| | Weight Percent Composition on the Dried Basis | | | | | |
|---|---|---|---|---|---|---|
| Sample No. | A | B | C | D | E | F |
| Iron | 11.8 | 11.4 | 11.9 | 11.1 | 12.0 | 12.0 |
| Pyrophosphate | 10.1 | 8.3 | 4.7 | 9.1 | 9.2 | 10.1 |
| Citrate | 34.3 | 35.9 | 44.2 | 37.6 | 35.8 | 36.5 |
| Phosphate | 16.8 | 17.1 | 12.9 | 15.8 | 17.4 | 16.3 |
| Sodium | 16.0 | 16.1 | 16.6 | 16.2 | 16.4 | 16.2 |
| Sulfate | 12.6 | 16.4 | 15.9 | 19.5 | 14.6 | 4.2 |

Any of the methods of the invention may be carried out with water-soluble ferric pyrophosphate citrate chelate compositions, having, by weight, from about 7% to about 11% iron, from about 14% to about 30% citrate, from about 10% to about 20% pyrophosphate and about 2% or less phosphate. The chelate compositions may have, by weight, about 1.5% phosphate or less, or about 1% phosphate or less. In an aspect of the invention, the chelate compositions have, by weight, about 0.1% phosphate or less.

U.S Patent No. 7,816,404, incorporated by reference herein unites entirely provides methods of preparing SFP-citrate chelate compositions in accordance with GMP standards (GMP-SFP). Any of the methods of the invention may be carried out with water-soluble ferric pyrophosphate citrate chelate compositions, having, by weight, from about 7% to about 11% iron, at least 14% citrate, at least 10% pyrophosphate. The chelate compositions may have, by weight, about 1.5% phosphate or less, or about 1% phosphate or less. In an aspect of the invention, the chelate compositions have, by weight, about 0.1% phosphate or less.

### Dialysis and Solutions

The term "dialysis" includes both hemodialysis and peritoneal dialysis and is defined as the movement of solute and water through a semipermeable membrane (the dialyzer) which separates the patient's blood from a cleansing solution (the dialysate). It is a clinical treatment procedure by which metabolic by-products, toxins, and excess fluid are removed from the blood of a subject with chronic kidney disease (CKD) by transfer across a dialysis membrane. Dialysis may be conventionally performed as hemodialysis, in which a synthetic membrane constitutes the dialysis membrane, or as peritoneal dialysis, in which a patient's peritoneal membrane constitutes the dialysis membrane. The patient's plasma tends to equilibrate with the dialysate solution over time. The composition of the dialysate permits one to remove, balance or even infuse solutes from and into the patient. The electrochemical concentration gradient is the driving force that allows the passive diffusion and equilibration between the dialysate and the patient's blood compartment. Dialysis-related iron deficiency affects about 90% of CKD patients by six months of treatment. The invention provides for method of administering SFP to patients suffering from CKD and undergoing dialysis via parenteral delivery including within hemodialysis solution or within peritoneal dialysis solution.

Hemodialysis refers to the use of a hemodialyzer to remove certain solutes from blood by virtue of their concentration gradients across a semipermeable membrane. The hemodialyzer, also referred to as an artificial kidney, is an apparatus by which hemodialysis is performed, blood being separated by the semipermeable membrane from a solution of such composition as to secure diffusion of certain solutes from the blood. The hemodialyzer can be used for ultrafiltration by which differences in fluid pressure bring about filtration of a protein-free fluid from the blood. Hemodialysis includes acute hemodialysis and maintenance hemodialysis.

Maintenance hemodialysis refers to long-term hemodialysis therapy for treatment of end stage renal failure. Patients on maintenance hemodialysis have been estimated to lose about 2 to 3 grams of iron per year, corresponding to approximately 6 ml per day (2 liters per year) blood loss from all sources (Eschbach et al. Ann. Intern Med. 87(6): 710-3, 1977).

During peritoneal dialysis, a patient's peritoneal membrane is used to exchange solutes and fluid with the blood compartment. Therefore, peritoneal dialysis is the treatment of uremia by the application of kinetic transport of water-soluble metabolites by the force of diffusion and the transport of water by the force of osmosis across the peritoneum. The peritoneum is the largest serous membrane of the body (approximately 2 m² in an adult). It lines the inside of the abdominal wall (parietal peritoneum) and the viscera (visceral peritoneum). The space between the parietal and visceral portions of the membrane is called the "peritoneal cavity." Aqueous solutions infused into the cavity (dialysate) contact the blood vascular space through the capillary network in the peritoneal membrane. The solution infused into the peritoneal cavity tends to equilibrate with plasma water over time and it is removed at the end of one exchange after partial or complete equilibration. The composition of the dialysate allows for the removal, balance or infusion of solutes from and into the patient. The electrochemical concentration gradient is the driving force that allows the passive diffusion and equilibration between the dialysate and blood compartment.

In general, the pharmaceutical compositions of this invention can be prepared by conventional techniques, as are described in Remington's Pharmaceutical Sciences, a standard reference in this field (Gennaro A R, Ed. Remington: The Science and Practice of Pharmacy. 20th Ed. Baltimore: Lippincott, Williams & Williams, 2000). For therapeutic purposes, the active components of this invention are ordinarily combined with one or more excipients appropriate to the indicated route of administration. A "dialysate solution or dialysate" is the solution used, on the opposite side of the membrane from the patient's blood, during dialysis. Dialysate is conventionally provided for use in either peritoneal dialysis (in which the peritoneal membrane constitutes the dialysis membrane) or hemodialysis (in which a synthetic membrane constitutes the dialysis membrane). Hemodialysate is generally prepared from two dry powder concentrates, including acid ("A") and base ("B") concentrates, which are reconstituted in treated water before use, or from two aqueous concentrates. The A concentrate, containing an organic acid and electrolytes and osmotic agents other than bicarbonate, is mixed with B concentrate containing bicarbonate and treated water in a dialysis machine to make the final hemodialysate. Peritoneal dialysate is a premixed solution of osmotic agents, electrolytes, and water that is used in dialysis without further constitution.

Presently, hemodialysis machines utilize an automated proportioning system to mix salts in deionized water in specific proportions to generate the final dialysate solution. The dialysate concentrates are usually supplied by the manufacturer either as a solution ready to use or as a premixed powder that is added to purified water in large reservoirs. The concentrates are pumped into a chamber in the dialysis machine where they are mixed with purified water to make the final dialysate solution.

Generally, the ionic composition of the final dialysate solution for hemodialysis is as follows: Na⁺ 132-145 mmol/L, K⁺ 0-4.0 mmol/L, Cl⁻ 99-112 mmol/L, Ca⁺⁺ 2.0-3.5 mEq/L, Mg⁺² 0.25-0.75 mmol/L, Dextrose 100-200 mg/dL. The correction of metabolic acidosis is one of the fundamental goals of dialysis. In dialysis, the process of H⁺ removal from the blood is mainly achieved by the flux of alkaline equivalents from the dialysate into the blood, thereby replacing physiological buffers normally utilized in the chemical process of buffering. In dialysis practice, base transfer across the dialysis membrane is achieved by using acetate or bicarbonate containing dialysate. In "Bicarbonate dialysis" the dialysate contains 27-35 mmol/L of bicarbonate and 2.5-10 mmol/L of acetate. On the other hand, in "Acetate dialysis" the dialysate is devoid of bicarbonate and contains 31-45 mmol/L of acetate. Ferric pyrophosphate is compatible with both acetate and bicarbonate based hemodialysis solutions.

The peritoneal dialysis fluid usually contains Na⁺ 132-135 mmol/L, K⁺ 0-3 mmol/L, Ca⁺⁺ 1.25-1.75 mmol/L, Mg⁺⁺ 0.25-0.75 mmol/L, Cl⁻ 95-107.5 mmol/L, acetate 35 mmol/L or lactate 35-40 mmol/L and glucose 1.5-4.25 gm/dL.

### Patient populations

The methods of the invention may be carried out on subjects suffering from anemia, subjects undergoing dialysis or both. The term "anemia" refers to a condition when the number of red blood cells and/or the amount of Hgb found in the red blood cells is below normal, and may be acute or chronic. For example, the term "anemia" includes but is not limited to iron deficiency anemia, renal anemia, anemia of chronic diseases/inflammation, pernicious anemia such as macrocytic achylic anemia, juvenile pernicious anemia and congenital pernicious anemia, cancer-related anemia, chemotherapy-related anemia, radiotherapy-related anemia, pure red cell aplasia, refractory anemia with excess of blasts, aplastic anemia, X-lined siderobalstic anemia, hemolytic anemia, sickle cell anemia, anemia caused by impaired production of ESA, myelodysplastic syndromes, hypochromic anemia, microcytic anemia, sideroblastic anemia, autoimmune hemolytic anemia, Cooley's anemia, Mediterranean anemia, Diamond Blackfan anemia, Fanconi's anemia and drug-induced immune hemolytic anemia . Anemia may cause serious symptoms, including hypoxia, chronic fatigue, lack of concentration, pale skin, low blood pressure, dizziness and heart failure.

Normal Hgb ranges for humans are about 14-18 g/dl for men and 12-16 for women g/dl with the average Hgb value for men at about 16 g/dL and for women at about 14 g/dL. Anemia may be considered a drop of Hgb levels below about 12 g/dL and severe anemia may be considered a drop in Hgb below about 8 g/dL. The grading system for anemia is provided in Table 3 below.

**Table 3 Grading System for Anemia* (Groopman & Itri, J. Natl. Cane. Inst. 91(19): 1616-1634. 1999)**

| Severity | WHO | NCI |
|---|---|---|
| Grade 0 (WNL)^{#} | ≥ 11.0 g/dL | WNL |
| Grade 1 (mild) | 9.5-10.9 g/dL | 10.0 g/dL to WNL |
| Grade 2 (moderate) | 8.0-9.4 g/dL | 8.0- 10 g/dL |
| Grade 3 (serious/severe) | 6.5-7.9 g/dL | 6.5-7.9 g/dL |
| Grade 4 (life threatening) | <6.5g/dL | <6.5g/dL |

| | | |
|---|---|---|
| * WHO = World Health Organization; NCI = National Cancer Institute WNL = within normal limits. # WNL hemoglobin values are 12.0-16.0 g/dL for women and 14.0-18.0 g/dL for men. | | |

Anemia may be assessed by assays well-known in the art such as a Complete Blood Count (CBC) test that measures the red blood cell (RBC) count, hematocrit, Hgb levels, white blood cell count (WBC), differential blood count, and platelet count. The first three parameters, the number of RBCs, hematocrit, and hemoglobin levels are the most commonly used in determining whether or not the patient is suffering from anemia.

[Total iron binding capacity (TIBC) measures the level for transferrin in the blood. Transferrin is a protein that carries iron in the blood and a higher than normal TIBC value is a sign of iron-deficiency anemia and a lower than normal level indicates anemia of inflammation, pernicious anemia, or hemolytic anemia. Additionally, tests for anemia include reticulocyte count, serum ferritin, serum iron, transferrin saturation, reticulocyte Hgb, percentage of hypochromic RBCs., direct or indirect Coombs' test, indirect bilirubin levels, serum haptoglobin, vitamin B 12 levels, folate levels, and urine Hgb. The upper normal limit of reticulocytes (immature red blood cells) is about 1.5%, a low count suggests problems with the bone marrow and a high count suggests hemolytic anemia (e.g., the patient's body is attempting to make up for a loss of RBCs).

The anemic subject may have undergone or is currently undergoing cancer therapies (e.g. chemotherapy and radiation), bone marrow transplant hematopoietic stem cell transplant, exposure to toxins, hemodialysis, peritoneal dialysis, treatment with parenteral nutrition, gastrectomy surgery and/or pregnancy.

ESA-resistant anemia or hyporesponsiveness to erythropoietin refers to the presence of at least one of the following conditions: i) a significant decrease in Hgb levels at a constant dose of ESA treatment, ii) a significant increase in the ESA dose requirement to achieve or maintain a certain Hgb level, iii) a failure to raise the Hgb level to the target range despite the ESA dose equivalent to erythropoietin greater than 150 IU/kg/week or 0.75 mg/kg/week of darbepoeitn-alpha or continued need for such high dose of ESA to maintain the target Hgb level. The method of the invention may be particularly useful in maintaining and/or increasing Hgb levels in subjects suffering from ESA-resistant anemia.

Anemia of inflammation is a type of anemia that commonly occurs with chronic, or long-term, illnesses or infections. The cytokines produced due to inflammation interfere with the body's ability to absorb and use iron and anemia results. In addition, cytokines may also interfere with the production and normal activity of erythropoietin. Hepcidin is thought to play a role as a key mediator of anemia of inflammation. The subject of any of the methods of the invention may be suffering from anemia of inflammation secondary to underlying disease state that is one of the following: chronic kidney disease, cancer, infectious diseases such as tuberculosis, HIV, endocarditis (infection in the heart), osteomyelitis (bone infection), hepatitis, inflammatory diseases such as rheumatoid arthritis and, lupus erthematosis, diabetes, heart failure, degenerative joint disease, and inflammatory bowel disease (IBD). IBD, including Crohn's disease, can also cause iron deficiency due to poor absorption of iron by the diseased intestine and bleeding from the gastrointestinal tract.

### Increased Ferritin Levels

CKD patients have increased iron losses due to chronic bleeding, frequent phlebotomy and blood trapping in the dialysis apparatus; and these patients have impaired dietary iron absorption Therefore, intravenous iron is commonly administered to hemodialysis patients because of the impaired dietary absorption (Babitt & Lin, J. AM. Soc. Nephrol. 23: 1631, 1634, 2012). CKD patients also have functional iron deficiency characterized by impaired iron release from body stores that is unable to meet the demand for erythropoiesis (reticulo-endothelial blockade). Thus, CKD patients may have normal or high serum ferritin levels and the treatment with IV iron is likely to have poor effectiveness and has the potential for adverse effects including liver iron overload (Vaziri, Am J. Med. 125(10);951-2, 2012), oxidant-mediated tissue injury due to excess iron deposition and increased risk of infection. Furthermore, CKD patients have an increase in hepcidin, the main hormone responsible for maintaining systemic iron homeostatis. It is predicted that in patients with excess hepcidin, administration of IV iron would have limited effectiveness because much of the iron is taken up by the liver and sequestered (Rostoker, Am J. Med. 125(10):991-999, 2012), and the remainder would be taken up by the red blood cells and would be recycled ineffectively. Plus, IV iron administration would further increase hepcidin levels. (Babitt & Lin, J. AM. Soc. Nephrol. 23: 1631, 1634, 2012)

While the methods of the invention provide for reducing or eliminating the dose of ESA to maintain Hgb levels in subjects suffering from anemia, these methods also reduce the dose of intravenously administered iron in subjects suffering from anemia. SFP when delivered directly into the circulation by any parenteral route binds directly to circulating iron carrier protein apotransferrin thereby forming monoferric or diferric transferrin, which then delivers iron directly to the red blood cell precursors in the bone marrow, bypassing processing by the reticuloendothelial macrophages and hepatocytes. Therefore, SFP increases or maintains the Hgb levels without increasing the iron stores (ferritin levels) in the subject and is an effective treatment of anemia of inflammation, a major contributor to the anemia seen in CKD patients.

Intravenous iron therapy does have risks and disadvantages. Anaphylactoid reactions have been reported in patients (Hamstra et al., J. Am. Med. Assoc. 243: 1726-31, 1980; Kumpf et al., DICP 24(2): 162-6, 1990). In addition, intravenous iron therapy can also cause hypotension, and loin and epigastric pain during dialysis, which may be severe enough to stop the treatment. Further, the intravenous drug is expensive and requires pharmacy and nursing time for administration. With intravenous iron therapy, serum iron, transferrin and ferritin levels have to be regularly monitored to estimate the need for iron and to measure a response to the therapy. Iron excess in dialysis patients refers to serum ferritin level of more than 500 µg/liter or more than 800 µg/liter or more than 1000 µg/liter. In addition, to the adverse affects listed above, excess ferritin levels may result in ESA resistance. Finally, there is also a concern about potential iron overload with intravenous therapy, since the risk of infection and possibly cancer are increased in patients with iron overload (Weinberg, Physiol. Rev. 64(1): 62-102, 1984).

As demonstrated in the clinical study provided in Example 1, administration of SFP increases or maintains Hgb level in subjects suffering from anemia while not increasing serum ferritin levels. Thus, the methods of the invention are useful for reducing the dose of intravenous iron administered to subjects suffering from anemia such as those undergoing dialysis or those suffering from CKD.

### EXAMPLES

### Example 1

### SFP delivered via the hemodialysate in hemodialysis dependent CKD is Safe and Effective

A prospective, randomized, double-blinded, multicenter, parallel control group clinical trial to determine the safety and efficacy of physiological iron maintenance in ESRD subjects by delivery of SFP via hemodialysate was carried out. The anemia manager making decisions about ESA dose changes and IV iron administration, the sponsor and independent site monitor were blinded to treatment group assignment. The primary objective of this study was to compare the safety and efficacy in sparing the need for supplemental IV iron required to maintain Hgb ≥10 g/dL in patients on 110 µg SFP-iron per liter of hemodialysate vs. conventional dialysate over 36 weeks. Conventional dialysate refers to hemodialysis without the addition of SFP to the dialysate.

The primary endpoint for efficacy was the amount of therapeutic iron needed and for safety, adverse events, vital signs, and clinical laboratory tests. Secondary endpoints of efficacy included Hgb levels, ESA doses and ESA response index.

The main inclusion criteria included adult ESRD patients on hemodialysis 3 times weekly, with an average Hgb 10 to 12 g/dL over the prior 2 months, that required any amount of IV iron over the prior 2 months, and having serum ferritin <800 µg/L with TSAT <40%. The main exclusion criteria were transferrin saturation (TSAT) ≤25% with ferritin ≤200 µ/dL, IV iron requirements of >500 mg over any consecutive 4 weeks in the prior 2 months, able to maintain TSAT >25% and ferritin >200 µg/L without IV iron in prior 2 months, epoetin dose >30,000 units/week or darbepoetin >60 µg/week or active infection.

Subjects were randomized equally to receive hemodialysis using dialysate solutions with SFP dialysate, or conventional dialysate at every dialysis session, for a total duration of 36 weeks plus a one-week follow-up. Hgb and serum iron parameters (TSAT and serum ferritin) were measured every 4 weeks and used to dose ESA, IV iron (Venofer®), and dialysate iron based on standardized criteria, with the goal of maintaining Hgb in the range of 11 to 12 g/dL, preferably 10.5 to 11.5 g/dL. Subjects in both groups were eligible to receive therapeutic doses of IV iron in order to maintain an iron replete state, with targets of TSAT 20% to 40% and ferritin 200 to 500 µg/L. SFP dosing was to be held and replaced with standard dialysate if it was confirmed that ferritin >800 µg/L, pre-dialysis TSAT >40%, or post-dialysis TSAT >100%. Safety parameters assessed included adverse events (AEs), physical examinations, vital signs, ECGs, and laboratory analyses (coagulation parameters, complete blood count, and blood chemistries including liver function tests).

### Patient Enrollment, Demographics, and Baseline Characteristics

Of the 11 patients enrolled, 64% were women and 36% were men, and 55% were Black and 45% were Hispanic. The patients ranged in age from 40 to 67 years, with a mean of 54 years and median of 57 years.

**Table 4: Baseline Characteristics**

| **Baseline Parameter** | **Mean** | **SD** |
|---|---|---|
| Hgb (g/dL) | 11.7 | 0.68 |
| Ferritin (mcg/L) | 509 | 129 |
| TSAT (%) | 24 | 5.4 |
| TIBC | 231 | 28 |
| Transferrin | 182 | 22 |
| Highest ESA Dose/Week in Prior Two Months (U) | 14,190 | 7,786 |
| Total IV Iron in Prior Two Months (mg) | 218 | 117 |

### Safety Findings [for all 11 Patients enrolled in the study]

SFP hemodialysate at the dose of 110 µg SFP-iron per liter was well tolerated, with the observed adverse effects (AE) consistent with a profile of clinical signs and symptoms generally observed to be common in the ESRD patient population on chronic hemodialysis. No patient was withdrawn from this study for a reason of an AE. There were no deaths on study. The data do not suggest any apparent SFP toxicity

### Efficacy Findings [for the 8 patients who completed the study, N=4 patients per group]

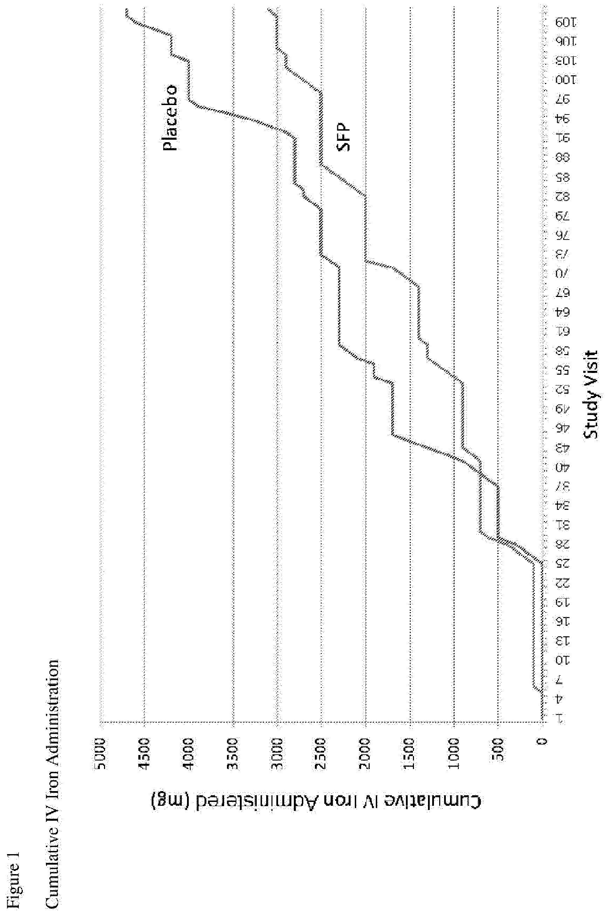

Over the duration of the study, the estimated mean weekly dose of IV iron administered was 21.5 mg for SFP compared to 32.6 mg for placebo, a 34% reduction, as shown in Figure 1.

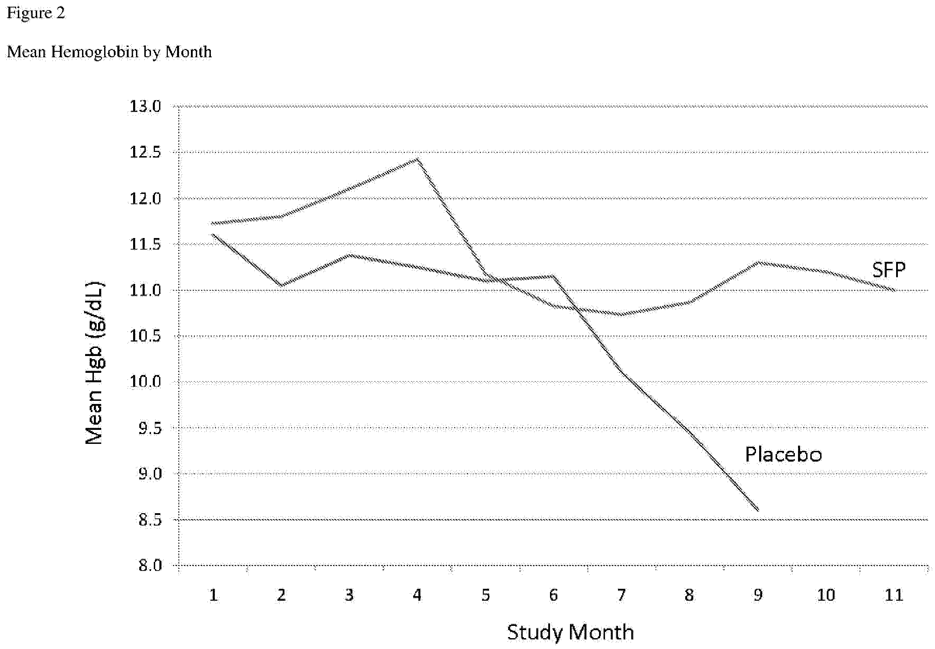

The mean Hgb in the subjects by month on study, as shown in Figure 2, demonstrates early separation of the curves, with the placebo group patients experiencing a notable and progressive decrease in Hgb over time.

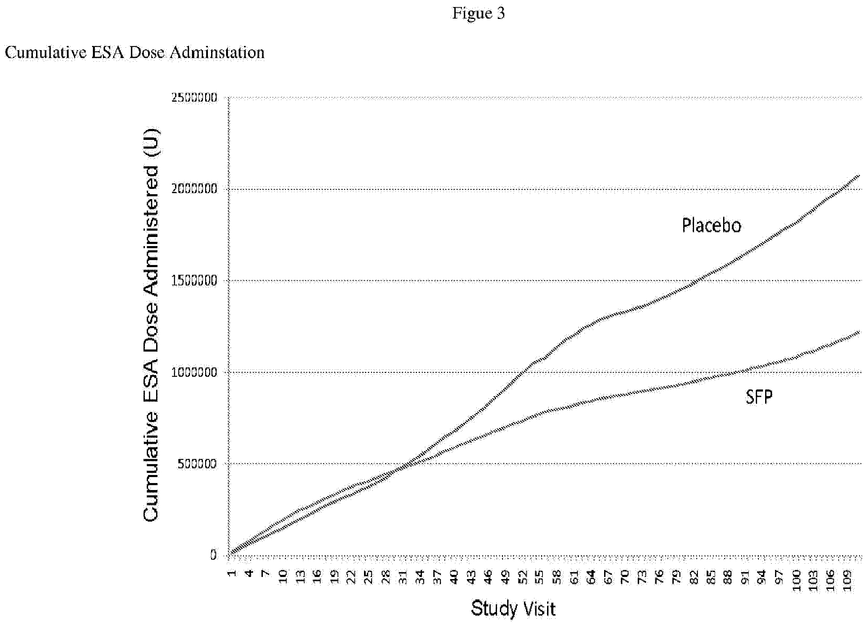

Over the duration of the study, the estimated mean weekly dose of ESA administered was 8,449 U for SFP compared to 14,384 U for placebo, a 41% reduction, as shown in Figure 3. For the final month on study, for the SFP treatment group the mean ESA dose was 53.5% lower and the median ESA dose was 50.5% lower than the ESA dose for placebo. Therefore, the data from this study showed an approximately 50% reduction in ESA dose requirements as compared to placebo.

This study demonstrates highly significant ESA sparing by SFP in CKD-HD patients without any increase in tissue iron stores or inflammation. This highly unexpected result is likely to be due to the unique mode of action of SFP. It is now increasingly recognized that a major contributor to anemia in CKD patients is the *anemia of inflammation* that is due to disordered iron homeostasis largely mediated by the peptide hepcidin (Yilmaz et al. Blood Purif. 32:220-5, 2011). Hepcidin excess likely accounts for impaired dietary iron absorption and impaired release of iron from reticulo-endothelial iron stores. Administration of SFP appears to supply iron without worsening the anemia of inflammation. Without being bound by theory, SFP binds directly to circulating iron carrier protein apotransferrin (apo-Tf) thereby forming monoferric or diferric transferrin (Tf), which then delivers iron directly to the red blood cell precursors in the bone marrow, bypassing processing by the reticuloendothelial macrophages and hepatocytes. When delivered to hemodialysis patients on a regular basis with every hemodialysis session 3-6 times a week SFP will generate mono and diferric Tf frequently. Coulon et al. (Nat. Med. 17(11): 1456-65, 2011) have demonstrated that Tf receptor, TfR1, plays an important role in erythropoiesis, besides the transport of Tf-bound iron into erythroid progenitors. TfR1 engagement by diferric Tf (Fe2-Tf) increases cell sensitivity to erythropoietin by inducing activation of mitogen-activated protein kinase and phosphatidylinositol 3-kinase signaling pathways. Fe2-Tf could act on TfR1 to promote robust erythropoiesis in both physiological and pathological situations, which may be relevant to SFP iron administration.

The data show a reduced requirement for ESA for maintaining Hgb levels after parenteral administration of SFP via the dialysate in CKD patients. It is expected that these findings apply to SFP delivered by any parenteral route in a variety of disease conditions. Parenteral routes for effective SFP delivery include but are not limited to intravenous, intramuscular, subcutaneous, intradermal, transdermal, via hemodialysis when added to hemodialysis solutions, via peritoneal dialysis when added to peritoneal dialysis solutions, or in conjunction with parenteral nutrition admixtures when added to parenteral nutrition admixture.

### Example 2

A 60 year old Caucasian male with hemodialysis dependent CKD and history of diabetes, hypertension, acute myocardial infarction, cerebrovascular accident and toe gangrene is receiving 400 units of recombinant erythropoietin per kilogram body weight per week to maintain Hgb in the 9-11 g/L target range. Patient has been receiving intravenous iron in a dose of 50 mg per week and is presumably iron replete as indicated by a serum ferritin level of 750 µg/L and a transferrin saturation of 25%. Intravenous iron is discontinued and the patient is started on SFP administration via the hemodialysate in a dose of 2 µmoles of SFP iron per liter of hemodialysate. There is progressive decline in ESA dose after 2 months of SFP therapy, and after 6 months patient is requiring only 100 units of recombinant erythropoietin per kilogram body weight per week to maintain Hgb in the 9-11 g/L target range. The serum ferritin at 6 months is 700 µg/L and transferrin saturation is 22%.

### Example 3

A 30 year old Hispanic male with hemodialysis dependent CKD secondary to glomerulonephritis is receiving 100 units of recombinant erythropoietin per kilogram body weight per week to maintain Hgb in the 9-11 g/L target range. Patient has been receiving intravenous iron in a dose of 100 mg every 2 weeks and is presumably iron replete as indicated by a serum ferritin level of 600 µg/L and a transferrin saturation of 27%. Intravenous iron is discontinued and the patient is started on SFP administration via the hemodialysate in a dose of 2 µmoles of SFP iron per liter of hemodialysate. There is progressive decline in ESA dose after 2 months of SFP therapy such that at 4 months recombinant erythropoietin is discontinued. At 12 months patient continues to maintain Hgb without ESA therapy while the serum ferritin is 650 µg/L and transferrin saturation of 33%.

### Example 4

An outpatient hemodialysis facility has a total of 120 patients. All patients are administered 50 mg intravenous iron sucrose once a week, in order to maintain iron balance, as long as the serum ferritin level is under 500 µg/L and transferrin saturation is under 50%. The 60 patients on the Monday- Wednesday-Friday schedule are switched from intravenous iron sucrose administration to slow continuous intravenous infusion of 10 mg SFP-iron with every hemodialysis session 3 times a week. The 60 patients on the Tuesday-Thursday-Saturday dialysis schedule continue to receive regular doses of intravenous iron. After 6 weeks a progressive decline in ESA doses is seen in the SFP group compared to the IV iron group. After 6 months the patients in the SFP group are requiring on an average 60% less ESA dose compared to their baseline dose. Furthermore, at 6 months the average dose in SFP group is 55% lower than the average dose in IV iron group.

### Example 5

An outpatient hemodialysis facility has a total of 100 patients. The 50 patients on the Monday- Wednesday- Friday schedule are switched from regular intravenous iron administration to SFP administration via the hemodialysate in a dose of 2 µmoles of SFP iron per liter of hemodialysate with every hemodialysis session 3 times a week. The 60 patients on the Tuesday-Thursday-Saturday dialysis schedule continue to receive regular doses of intravenous iron. After 8 weeks a progressive decline in ESA doses is seen in the SFP group compared to the IV iron group. After 6 months the patients in the SFP group are requiring on an average 50% less ESA dose compared to their baseline dose. Furthermore, at 6 months the average dose in SFP group is 55% less than the average dose in IV iron group.

Accordingly, the invention is characterized by the following features:
1. A method of treating iron deficiency that reduces or eliminates the dose of an erythropoiesis stimulating agent (ESA) for achieving or maintaining target hemoglobin levels in a subject suffering from anemia comprising
   (a) administering to the subject a dose of a soluble ferric pyrophosphate composition (SFP) effective to increase hemoglobin levels of the subject, and
   (b) after SFP administration, (i) administering to the subject a dose of ESA that is at least 15% less than the dose of ESA administered prior to SFP administration, or (ii) discontinuing ESA administration.
2. A method of delaying or preventing iron deficiency and delaying or preventing the need for administration of a dose of an erythropoiesis stimulating agent (ESA) for achieving or maintaining target hemoglobin levels in a subject suffering from iron loss or iron deficiency comprising
   (a) administering to the subject a therapeutically effective does of soluble ferric pyrophosphate composition (SFP) effective to increase hemoglobin levels of the subject, and
   (b) delaying or preventing the administration of ESA, wherein the subject is not currently receiving ESA to achieve or maintain target hemoglobin levels.
3. The method of feature 1 or 2 wherein the dose of SFP is administered via a parenteral route selected from the group consisting of intramuscular, subcutaneous, intravenous, intradermal, transdermal, transbuccal, sublingual, intra-peritoneal, in
   conjunction hemodialysis or in conjunction with peritoneal dialysis.
4. The method of feature 1 or 2, wherein the dose of SFP is administered orally.
5. The methods of any of the preceding features wherein the dose of SFP is administered in conjunction with other drugs such as heparin or parenteral nutrition admixtures or dialysis solutions.
6. The method of any of the preceding features, wherein the dose of SFP increases or maintains the hemoglobin level of the subject at 9 g/dL or greater.
7. The method of any one of features 1-3 or 6, wherein the dose of SFP is administered via hemodialysate at a dose ranging from 90 µg Fe/L dialysate to 120 µg Fe/L dialysate.
8. The method of feature 7, wherein the SFP is administered via hemodialysate at a dose of 110 µg Fe/L dialysate.
9. The method of any one of features 1-6, wherein the dose of SFP is administered via infusion or intravenous injection at a dose ranging from 2.4 mg to 48 mg iron per day at a rate of 0.1 to 2 mg iron per hour.
10. The method of any one of features 1-6 wherein the dose of SFP is administered into the circulation at a dose ranging 2.4 mg to 48 mg iron per day at a rate of 0.1 to 2 mg iron per hour.
11. The method of any one of the preceding features, wherein the dose of ESA administered to the subject after SFP administration is at least 25% less than the dose of ESA administered prior to SFP administration.
12. The method of any of the preceding features, wherein the dose of ESA administered to the subject after SFP administration is at least 40% less than the dose of ESA administered prior to SFP administration.
13. The method of any one of the preceding features, wherein the dose of ESA administered to the subject after SFP administration is 15% to 50% less than the dose of ESA administered prior to SFP administration.
14. A method of increasing hemoglobin levels in subjects suffering from anemia comprising administering soluble ferric pyrophosphate (SFP) to the subject by adding SFP to the hemodialysate in a concentration of 110 µg Fe/L.
15. A method of increasing hemoglobin levels in subjects suffering from anemia comprising administering 2.4 mg to 48 mg of soluble ferric pyrophosphate (SFP) iron per day at a rate of 0.1 to 2 mg iron per hour into the circulation of the subject by a route selected from the group consisting of oral, intramuscular, subcutaneous, intravenous, intradermal, transdermal, transbuccal, sublingual, intraperitoneal, in conjunction with heparin or in conjunction with parenteral nutrition admixtures, in conjunction with peritoneal dialysis solutions or in conjunction with hemodialysis solutions, wherein the dose administered to the patient is based on the bioavailability of SFP using the specific route of administration.
16. The method of feature 14 or 15, wherein the subject is receiving erythropoiesis stimulating agent (ESA) to achieve or maintain hemoglobin levels, the method further comprises administering a dose of ESA, after SFP administration, that is less than the dose of ESA administered prior to SFP administration.
17. The method of any of the preceding features wherein the SFP comprises iron chelated with citrate and pyrophosphate.
18. The method of any of the preceding features wherein the SFP comprises iron in an amount of 7% to 11% by weight, citrate in an amount of at least 14% by weight, and pyrophosphate in an amount of at least 10% by weight.
19. The method of any of the preceding features wherein the SFP is administered via dialysate and the dose of intravenously administered iron after SFP administration is at least 10% less than the dose of intravenously administered iron prior to SFP administration.
20. The method of any of the preceding features wherein the subject is suffering from chronic kidney disease, optionally stage III, IV or V.
21. The method of any of the preceding features wherein the subject is suffering from stage IV or V chronic kidney disease.
22. The method of any of the preceding features wherein the subject is undergoing hemodialysis.
23. The method of any of the preceding features wherein the subject is suffering from anemia of inflammation.
24. The method of any of the preceding features wherein the subject is suffering from infection, optionally chronic infection.
25. The method of any one of the preceding features wherein the subject is suffering from cancer, heart failure, autoimmune disease, sickle cell disease, thalassemia, blood loss, transfusion reaction, diabetes, vitamin B12 deficiency, collagen vascular disease, thrombocytopenic purpura, Celiac disease, endocrine deficiency state, hypothyroidism, Addison's disease, Crohn's Disease, systemic lupus erythematosis, rheumatoid arthritis, juvenile rheumatoid arthritis, ulcerative colitis, immune disorders, eosinophilic fasciitis, hypoimmunoglobulinemia, thrymoma/thymic carcinoma, graft vs. host disease, preleukemia, Nonhematologic syndrome, Felty syndrome, hemolytic uremic syndrome, myelodysplasic syndrome, nocturnal paroxysmal hemoglobinuria, osteomyelofibrosis, pancytopenia, pure red-cell aplasia, Schoenlein-Henoch purpura, malaria, protein starvation, menorrhagia, systemic sclerosis, liver cirrhosis, hypometabolic states, congestive heart failure, chronic infection, HIV/AIDS, tuberculosis, oseomyelitis, hepatitis B, hepatitis C, Epstein-bar virus, parvovirus, T cell leukemia virus, bacterial overgrowth syndrome, red cell membrane disorder, hereditary spherocytosis, hereditary elliptocytosis, red cell enzyme defects, hypersplenism, immune hemolysis or paroxysmal nocturnal hemoglobinuria.
26. The method of any one of the preceding features wherein the anemia is due to overt or functional iron deficiency.
27. The method of any one of the preceding features wherein dose of SFP is administered during hemodialysis within the hemodialysate solution.
28. The method of any of the preceding features wherein SFP is administered during peritoneal dialysis within the peritoneal dialysis solution.
29. The method of any one of the preceding features wherein SFP is administered with parenteral nutrition within parenteral nutrition admixture.
30. The method of any one of the preceding features wherein SFP is administered at a therapeutically effective dose that i) increases at least one marker of iron status selected from the group consisting of serum iron, transferrin saturation, reticulocyte hemoglobin, serum ferritin, reticulocyte count, and whole blood hemoglobin and ii) decreases or eliminates the need for ESA administration to achieve or maintain target hemoglobin levels, or the need for transfusion of whole blood, packed red blood cell or blood substitutes.
31. The method of feature 30, wherein the subject is suffering from non-anemic iron deficiency and administration of the therapeutically effective dose of SFP reduces fatigue, increases physical and cognitive ability, or improves exercise tolerance in the subject.
32. The method of any one of the preceding features wherein SFP is administered in a therapeutically effective dose that will reduce or abolish the clinical manifestations of restless leg syndrome associated with iron deficiency.

## Claims

1. A composition comprising soluble ferric pyrophosphate (SFP) formulated for administration by intravenous injection or intravenous infusion at a SFP iron dose ranging from 2.4 to 48 mg per unit dose.

2. The composition of claim 1, wherein the SFP iron dose ranges from 2.4-10 mg, from 5-10 mg, from 10-48 mg, or from 30-48 mg.

3. The composition of claim 1 or claim 2 wherein the composition is formulated for administration at a rate of 0.1 to 2 mg per hour or 1 to 2 mg hour.

4. The composition of any one of claims 1-3, wherein the SFP comprises iron chelated with citrate and pyrophosphate, wherein optionally the SFP comprises iron in an amount of 7% to 11% by weight, citrate in an amount of at least 14% by weight, and pyrophosphate in an amount of at least 10% by weight.

5. The composition of any one of claims 1-4 for use in the treatment of a subject suffering from chronic kidney disease, anemia, overt or functional iron deficiency.

6. The composition for use of claim 5, wherein the composition reduces or eliminates the need to administer an erythropoietin stimulating agent.

7. The composition for use of claim 6, wherein the composition is effective to delay or prevent the need to administer an erythropoiesis stimulating agent (ESA) to achieve or maintain target hemoglobin levels in the subject.

8. The composition for use of claim 6, wherein the composition is effective to reduce the need to administer the ESA such that the dose of ESA is a dose of 15%-50% less than the dose of ESA administered prior to the SFP administration.

9. The composition for use of claim 7 or claim 8, wherein the ESA is administered via oral delivery.

10. The composition for use of claim 7, wherein the target hemoglobin level is about 9-11 mg/dL and optionally wherein the composition is effective at avoiding iron overload.

11. The composition for use of any one of claims 5-10, wherein the SFP is administered during a hemodialysis session.

12. The composition for use of claim 5, wherein the anemia is iron deficiency anemia and the use increases at least one marker of iron status selected from the group consisting of serum iron, transferrin saturation, reticulocyte Hgb, serum ferritin, reticulocyte count, percentage of hypochromic red blood cells, and whole blood hemoglobin.

13. The use according to claim 12, wherein the method comprises (a) reducing or eliminating the need for blood transfusions of whole blood or packed red blood cells or blood substitutes in anemic subjects, or (b) decreasing or eliminating the need for ESA administration.

14. A medicament comprising the composition of any one of claims 1-4 and heparin or a parenteral nutrition admixture.
